**Europäisches Patentamt**

**European Patent Office**

**Office européen des brevets**

(19)

(11) Veröffentlichungsnummer : **0 480 172 B1**

(12) **EUROPÄISCHE PATENTSCHRIFT**

(45) Veröffentlichungstag der Patentschrift :
**20.10.93 Patentblatt 93/42**

(51) Int. Cl.⁵ : **A61K 7/16**

(21) Anmeldenummer : **91114887.2**

(22) Anmeldetag : **04.09.91**

(54) **Zahnpflegemittel.**

(30) Priorität : **09.10.90 DE 4031953**
**22.04.91 DE 4113044**

(43) Veröffentlichungstag der Anmeldung :
**15.04.92 Patentblatt 92/16**

(45) Bekanntmachung des Hinweises auf die
Patenterteilung :
**20.10.93 Patentblatt 93/42**

(84) Benannte Vertragsstaaten :
**AT BE CH DE DK ES FR GB GR IT LI LU NL SE**

(56) Entgegenhaltungen :
**FR-A- 2 625 676**
**CHEMICAL ABSTRACTS, Band 99, Nr. 26, 26.**
**Dezember 1983, Columbus, OH (US); N.DIMI-**
**TROVA et al., Seite 370, Nr. 218412b**
**MATERIALS CHEMISTRY AND PHYSICS,**
**Band 13, Nr. 1, 1985; G.CHAUVET et al., Seiten**
**503-516**
**CHEMICAL ABSTRACTS, Band 112, Nr. 26, 25.**
**Juni 1990, Columbus, OH (US); H.STRUSZC-**
**ZYK et al., Seite 110, Nr. 237262f**

(73) Patentinhaber : **Degussa Aktiengesellschaft**
**Weissfrauenstrasse 9**
**D-60311 Frankfurt (DE)**
Patentinhaber : **THE CHEMICAL**
**PHARMACEUTICAL RESEARCH INSTITUTE**
**(NIHFI)**
**1a, Climent Okhridski Blvd.**
**Sofia 1156 (BG)**

(72) Erfinder : **Again, Ivan, Prof.**
**Hubeha 84**
**Sofia 1156 (BG)**
Erfinder : **Boyanova, Vera, Prof.**
**Anjel Kocev 15**
**Sofia 1156 (BG)**
Erfinder : **Otto, Karin, Dr.**
**Corniceliusstrasse 4a**
**W-6450 Hanau 1 (DE)**
Erfinder : **Neumüller, Matthias**
**Lindenstrasse 18**
**W-6467 Hasselroth 1 (DE)**
Erfinder : **Dippmann, Harald**
**Gleiwitzer Strasse 15**
**W-6450 Hanau 9 (DE)**

(74) Vertreter : **Weber, Wolfgang**
**Degussa AG Fachbereich Patente**
**Rodenbacher Chaussee 4 Postfach 1345**
**D-63403 Hanau (DE)**

**Beschreibung**

Die Erfindung betrifft ein Zahnpflegemittel zur Entfernung des Zahnbelages, welches als aktiv-prophylaktisches, hygiene-kosmetisches und heilendes Präparat bei der alltägliche Benutzung und in der stomatologischen Praxis Anwendung findet.

Es ist bekannt, daß Cellulosepulver mit einer Partikelgröße von 1 bis 350 Mikrometer für ein direktes Tablettieren von Arzneiformen eingesetzt werden kann. Dabei werden zusätzliche Hifsmaßnahmen, sowie die Benutzung von zusätzlichen Hilfsstoffen auf Grund der spezifischen rheologischen Parametern der pulverförmigen Zellulose eingespart.

Bei der Herstellung von Hygiene- und Prophylaxemitteln der Mundhöhle, wie es die alltäglich gebrauchten Zahnpflegemittel mit unterschiedlichem aktiv-prophylaktischem und kosmetischem Effekt sind, ist die Auswahl von passenden Agentien, die effektiv den Zahnbelag beseitigen, von wesentlicher Bedeutung.

Zu diesem Zweck werden bekannterweise Putzkörper wie : Calciumcarbonat, Dicalciumphosphat-dihydrat, u. a. benutzt, welche, obwohl sie dank ihrer mechanischen Einwirkung den Belag auf den Zähnen beseitigen, gleichzeitig zu einem unterschiedlichen Grad den harten Zahnschmelz beschädigen.

Es ist bekannt, mikrokristalline Zellulose, wie zum Beispiel AVICEL PH 105, als Abrasivkomponente in Zahnpasten einzusetzen (FR-A 2 625 676).

Aufgabe der Erfindung ist die Verwendung eines effektiven, belagreinigenden Agens in für die Mundhöhle bestimmten Mitteln, welche keine unerwünschten Nebenwirkungen auf den harten Zahnschmelz hervorrufen und den normalen physiologischen Zustand der Mundhöhle erhalten.

Gegenstand der Erfindung ist ein Zahnpflegemittel bestehend aus den üblichen bekannten Bestandteilen eines Zahnpflegemittels, welches dadurch gekennzeichnet ist, daß es anstelle von bekannten Abrasivkomponenten als Putzkörper Cellulosepulver mit den folgenden Merkmalen:

| Struktur | Pulver | |
|---|---|---|
| Teilchengröße in Mikron ca. | 40 - 150 | |
| Schüttgewicht bei Absackung g/l | 220 - 230 | |
| pH-Wert | 5,0 - 7,5 | |
| Brechungsindex | 1,55 | |
| Dichte g/cm$^3$ | ca. 1,5 | |
| Trocknungsverlust 2 h/105 °C | < 6 | % |
| Glührückstand 2 h/850 °C | < 0,3 | % |
| wasserlösliche Bestandteile | < 1,0 | % |
| ätherlösliche Bestandteile | < 0,15 | % |
| Calcium-Ionen | < 0,05 | % |
| Chlorid-Ionen | < 0,05 | % |
| Sulfat-Ionen | < 0,05 | % |
| Schwermetall-Ionen als Pb | < 10 ppm | |
| Zn | < 2 ppm | |
| As | < 1 ppm | |
| Cr | < 1 ppm | |
| Fe | < 10 ppm | |
| Cu | < 1 ppm | |

enthält.

Das Cellulosepulver kann in dem Zahnpflegmittel in einer Menge von 1 bis 25 Gew.-%, vorzusgweise von 10 bis 20 Gew.-%, enthalten sein.

Weitere zusätzliche Wirkstoffe, die Plaque entfernend oder Plaque verhindernd, beziehungsweise entzündungshemmend sind, können entfallen.

Bevorzugt kann man ein Cellulosepulver, das unter den Namen ELCEMA® P 050 und P 100 im Handel ist und die folgenden physikalisch-chemischen Parameter aufweist, verwenden:

Native, hochprozentige α-Cellulose, aus 1,4-β-glykosidisch verknüpften D-Glukosemolekülen, wobei n = vorwiegend um 500 und darüber liegt. Feines, weisses Pulver ohne Geschmack und Geruch

unlöslich in Wasser
unlöslich in verdünnten Säuren
Quellung in verdünnten Laugen
unlöslich in organischen Lösungsmitteln

| ELCEMA®-Typ | P 100 | P 050 |
|---|---|---|
| Struktur | Pulver | Pulver |
| Teilchengröße in Mikron ca. | 50 - 150 | 40 - 70 |
| Schüttgewicht bei Absackung g/l | ca. 220 | ca. 230 |
| pH-Wert | 5,0 - 7,5 | 5,0-7,5 |
| Brechungsindex | 1,55 | 1,55 |
| Dichte g/cm$^3$ | ca. 1,5 | ca. 1,5 |

| | | |
|---|---|---|
| Trocknungsverlust 2 h/105 °C | < 6 | % |
| Glührückstand 2 h/850 °C | < 0,3 | % |
| wasserlösliche Bestandteile | < 1,0 | % |
| ätherlösliche Bestandteile | < 0,15 | % |
| Calcium-Ionen | < 0,05 | % |
| Chlorid-Ionen | < 0,05 | % |
| Sulfat-Ionen | < 0,05 | % |
| Schwermetall-Ionen als Pb | < 10 | ppm |
| Zn | < 2 | ppm |
| As | < 1 | ppm |
| Cr | < 1 | ppm |
| Fe | < 10 | ppm |
| Cu | < 1 | ppm |

Identitätsnachweis

Die Substanz löst sich langsam in ammoniakalischer Kupferoxidlösung. Mit Jodlösung tritt in wässriger Dispersion keine Violett- oder Blaufärbung auf.

Das erfindungsgemäß einsetzbare Cellulosepulver wird in dem Deutschen Arzneibuch 9. Ausgabe 1986 (DAB9) auf Seite 621, in der USP XXII NF XVII (The United States Pharmacopeia, The National Formulary 1990, Seite 1916 sowie in der European Pharmacopoeia 2nd Edition Part. II Eighth Fascicule 1984, Seite 315 beschrieben.

Bei den durchgeführten Untersuchungen an erfindungsgemäßen Zahnpflegemitteln mit Cellulosepulver, das eine Größe der Partikel von 40 bis 150 Mikrometer aufweist, wird festgestellt, daß sie bei der Verwendung in Mengen von 1 bis 25 Gew.-%, vorzugsweise 10 bis 20 Gewichtsprozent in verschiedenen Zusammensetzungen von Zahnpflegemitteln, effektiv den Zahnbelag beseitigt, dessen Neubildung verhindert und entzündungshemmend in der Mundhöhle wirkt. Zudem besteht Verträglichkeit mit allen biologisch aktiven Komponenten wie: Fluorid-Ionen, Zinkionen, Chemietherapeutika, Enzymen, Komponenten von natürlicher Herkunft, welche gegebenenfalls zusätzlich zwecks höherer Inhibierung der Entwicklung des Zahnbelages verwendet werden und ebenfalls eine entzündungshemmende Wirkung aufweisen können.

Das Cellulosepulver ist chemisch inert und gleichzeitig physiologisch verträglich. Diese Eigenschaften ermöglichen ihre Verwendung in Zahnpflegerezepturen, deren Inhaltsstoffe mit bekannten Putzkörpern unverträglich sind. Es ist zum Beispiel bekannt, daß Fluorverbindungen ihre Wirkung nur für eine begrenzte Zeitspanne neben bekannten Putzkörpern wie Calciumcarbonat, Di-Calciumphosphat-di-hydrat u. a. behalten. Bei der Kombination des Cellulosepulvers mit Zahnpflegemitteln, die Fluorionen enthalten, werden Zahnpflegemittel formuliert, die die Aktivität des Fluors auf eine unbegrenzte Zeitspanne, mindestens jedoch 18 Monate, erhalten. Dies ermöglicht die Produktion von Zahnpflegemitteln ohne Berücksichtigung der bisher notwendigen Beschränkungen in der Lagerfähigkeit.

In einer bevorzugten Ausführungsform der Erfindung kann das Cellulosepulver in fluorhaltigen Zahnpflegemitteln eingesetzt werden.

Beispiele:

| Beispiel 1 | Gew.-% |
|---|---|
| ELCEMA® P 100 | 10,0 |
| Glyzerin | 20,0 |
| Aerosil® 200 | 1,0 |
| Blanose | 1,5 |
| Süßstoff | 1,0 |
| Konservierungsmittel | 0,5 |
| Aromastoff | 1,0 |
| Schaummittel | 1,0 |
| Wasser bis | 100 |

| Beispiel 2 | Gew.-% |
|---|---|
| ELCEMA® P 100 | 20,0 |
| Glyzerin | 10,0 |
| Aerosil® 200 | 1,0 |
| Blanose | 1,5 |
| Süßstoff | 1,0 |
| Konservierungsmittel | 0,5 |
| Aromastoff | 1,0 |
| Schaummittel | 1,5 |
| Wasser bis | 100 |

<u>Beispiel 3</u>                         Gew.-%

| | |
|---|---|
| ELCEMA® P 050 | 15,0 |
| Glyzerin | 20,0 |
| Aerosil® 200 | 3,0 |
| Blanose | 1,5 |
| Konservierungsmittel | 0,5 |
| Aromastoff | 1,0 |
| Süßstoff | 1,0 |
| Schaummittel | 1,5 |
| Wasser      bis | 100 |

Die in den Beispielen eingesetzten Stoffe können wie folgt charakterisiert werden:
Aerosil® 200 ist eine pyrogen hergestellte Kieselsäure mit einer BET-Oberfläche von $200 \pm 25$ m²/g.
Blanose ist eine Natrium-carboxymethylcellulose, die als Bindemittel eingesetzt wird.

Süßstoff ist Saccharin

Konservierungsmittel ist Nipagin und Nipasol, die zusammen 0,1 Gew.-% ausmach oder Natriumbenzoicum (0,5 Gew.-%).

Aromastoff ist Pfefferminzöl

Schaummittel ist Natriumlaurylsulfat ELCEMA® P 100 oder P 050 ist eine native, hochprozentige $\alpha$-Cellulose, aus 1,4-$\beta$-glykosidisch verknüpften D-Glukosemolekülen, wobei n = vorwiegend um 500 und darüber liegt.
Feines, weisses Pulver ohne Geschmack und Geruch
unlöslich in Wasser
unlöslich in verdünnten Säuren
Quellung in verdünnten Laugen
unlöslich in organischen Lösungsmitteln

| ELCEMA®-Typ | P 100 | P 050 |
|---|---|---|
| Struktur | Pulver | Pulver |
| Teilchengröße in Mikron ca. | 50-150 | 40 - 70 |
| Schüttgewicht bei Absackung g/l | ca. 220 | ca. 230 |
| pH-Wert | 5,0-7,5 | 5,0-7,5 |
| Brechungsindex | 1,55 | 1,55 |
| Dichte g/cm$^3$ | ca. 1,5 | ca. 1,5 |

| Trocknungsverlust 2 h/105 °C | < 6 | % |
|---|---|---|
| Glührückstand 2 h/850 °C | < 0,3 | % |
| wasserlösliche Bestandteile | < 1,0 | % |
| ätherlösliche Bestandteile | < 0,15 | % |
| Calcium-Ionen | < 0,05 | % |
| Chlorid-Ionen | < 0,05 | % |
| Sulfat-Ionen | < 0,05 | % |
| Schwermetall-Ionen als Pb | < 10 ppm | |
| Zn | < 2 ppm | |
| As | < 1 ppm | |
| Cr | < 1 ppm | |
| Fe | < 10 ppm | |
| Cu | < 1 ppm | |

Identitätsnachweis

Die Substanz löst sich langsam in ammoniakalischer Kupferoxidlösung. Mit Jodlösung tritt in wässriger Dispersion keine Violett- oder Blaufärbung auf.

Zur Prüfung der chemischen Reinheit von ELCEMA® können die Prüfmethoden folgender Pharmakopöen angewendet werden:

Deutsches Arzneibuch 9. Ausgabe 1986 (DAB9), Seite 621 USP XXII NF XVII (The United States Parmacopeia, The National Formulary) 1990, Seite 1916. European Pharmacopoeia 2nd Edition Part. II Eight Fascicule 1984, Seite 315.

Bei der klinischen Untersuchung des Zahnpflegemittels werden die folgenden Wirksamkeiten untersucht: Plaque-Entfernung, Plaque-Verhinderung und Entzündungshemmung.

Die Untersuchung wird an 40 Personen im Alter zwischen 25 und 35 Jahren durchgeführt. Diese Personen sind körperlich gesund, ohne Gesichts- und Kieferdeformation, ohne Karies, ohne Plomben auf den Zähnen, die untersucht werden, und mit gering ausgeprägter katarrhatischen Zahnfleischentzündung. Eine vorläufige hygienische Vorbereitung wird durch eine Entfernung von Plaque und Zahnstein durchgeführt. Die Untersuchung beginnt drei Tage nach dieser Vorbehandlung und 12 Stunden nach dem letzten Bürsten der Zähne. Die folgenden Werte werden bestimmt: OHI-S nach Green und Vermillion

PMA nach Massler und Schour
die Zahl der gewanderten Leucocyten, die abgeschuppten Epithelzellen, die Erythrocyten in der Mundflüssigkeit nach Jasinovski.

Die Kontrolle des Bürstens erfolgt nach den Instruktionen gemäß "Methoden für die klinische Studie von Zahnpasten" Moskau-Sofia 1980.

Die Untersuchung wird begonnen mit der Registrierung des Plaque-Index PL und dem Entzündungsindex GI.

Die Plaque-Entfernung und die Plaque-Verhinderung wird nach der folgenden Methode gemessen:

Die Test- und Kontrollgruppen putzen regelmässig die Zähne im Verlaufe von 4 Wochen mit Zahnpasta unter Verwendung von neuen Zahnbürsten. Die Zähne werden zweimal am Tage jeweils drei Minunten lang unter Aufsicht geputzt. Das abendliche Bürsten wird auf alle Zahnoberflächen mit intensiven horizontalen, vertikalen und kreisenden Bewegungen durchgeführt.

In jedem der 2 Abschnitte werden 10 bis 12 Bürsten benutzt:

| $\underline{876}$ | $\underline{54}$ | $\underline{321}$ | $\underline{123}$ | $\underline{45}$ | $\underline{678}$ |
|---|---|---|---|---|---|
| 876 | 54 | 321 | 123 | 45 | 678 |

Die Registrierung des Plaque Indexes wird zu Beginn des Tests (diagnosticalindex) an einem Zahn durchgeführt, der für eine Nacht nicht gebürstet wird, und am Ende einer jeden Woche. Die Plaque wird durch Lutschen einer Standard Farbtablette bis zur völligen Auflösung gefärbt. Der Plaque Index wird nach der Methode von Green Vermillion (einfacher Index) bestimmt.

Der Plaquebelag der

Vestribularoberflächen von $\frac{616}{1}$ wird ebenso wie der Belag der Zwischenräume sowie der Belag der auf der Zungenseite liegenden Oberflächen von $\overline{6} \quad \overline{6}$ Wert:

- 0 - keine Plaque festgestellt
- 1 - Plaque auf 1/3 der Zahnoberfläche
- 2 - Plaque auf 2/3 der Zahnoberfläche
- 3 - Plaque auf der vollständigen Zahnoberfläche

Die Plaque wird nach 1 bis 4 Wochen bestimmt in Prozent zu dem ursprünglichen Zustand.

Der Grad der Entzündung wird am 20 Testpersonen, die bei der Plaqueuntersuchung teilnehmen und eine leicht ausgeprägte, chronische Zahnfleischentzündung aufweisen, durchgeführt.

Es wird der PMA-Index am Anfang und nach 1 bis 4 Wochen bestimmt. Das Zahnfleisch wird mit $J_2$-KJ-Lösung nach der Methode von Svrakov-Pissarev behandelt und die Entzündung um $\frac{616}{616}$ entsprechend der folgenden Werte festgestellt:

- 0 - keine Entzündung beobachtet
- 1 - Zahnfleischpapilla entzündet

Der PMA-Index wird nach 1 bis 4 Wochen in Prozent auf den Ausgangszustand bezogen.

Die für das erfindungsgemäße Zahnpflegemittel ermittelten Werte werden mit den parallel festgestellten Werten von bekannten Zahnpflegemitteln verglichen. Dabei werden die folgenden Ergebnisse erzielt:

| Zahnpflegemittel mit Putzkörper | Plaque-entfernung | Plaque-verhin-derung | Entzün-dungs-hemmen-der Effekt in der Mund-höhle |
|---|---|---|---|
| 1. Calcium carbonat | 32,38 % | 11,03 % | 5 - 10 % |
| 2. Dicalcium phos-phat dihydrat (Benckiser-Hoechst) | 42,13 % | 10,18 % | 5 - 10 % |
| 3. Gefällte Kieselsäure (Sident® 9, Degussa) | 38,52 % | 10,15 % | 5 - 10 % |
| 4. Micro-kristalline Cellulose (Micricel) | 32,15 % | 5,12 % | 5 - 10 % |
| 5. Cellulose-pulver (Elcema®, Degussa) | 78,16 % | 20,04 % | 28 - 33 % |

Die Tabelle zeigt, daß das Zahnpflegemittel 5 mit ELCEMA® (Cellulosepulver gemäß Erfindung) eine überraschend hohe plaqueentfernende Wirkung im Vergleich zu den Zahnpflegemitteln 1, 2, 3 und 4 hat. Deutlich übertrifft das Zahnpflegemittel 5 auch das Zahnpflegemittel 4, welches mikrokristalline Zellulose (Micricel) gemäß dem Stand der Technik enthält.

Besonders überraschend sind die unerwartet hohen Werte für den plaqueverhindernden Effekt und den entzündungshemmenden Effekt. Zusätzlich tritt eine heilende Wirkung in der Mundhöhle ein.

Die in dem Zahnpflegemittel 4 eingesetzte mikrokristalline Cellulose Micricel ist produktgleich zu AVICEL. AVICEL ist eine mikrokristalline Cellulose, die durch Säurebehandlung von Cellulose vor der Sprühtrocknung teilweise gereinigt und depolymerisiert wurde (vgl. The National Formulary XIII Edition, Monograph 43).

Die Stabilitätsprüfung von Fluoridionen enthaltenden Zahnpflegemitteln, die Cellulosepulver als Abrasivkomponente enthalten, mit Hilfe von beschleunigten Alterungsmethoden und unter Anwendung der Arhenius-Gleichung ergibt, daß in Zahnpflegemitteln mit Natriumfluorid und mit Natriummonofluorphosphat die aktiven Fluor- und Monofluorphosphat-Ionen ihre Quantität für einen Zeitraum von mehr als 3 bis 4 Jahren unverändert beibehalten.

**Patentansprüche**

1. Zahnpflegemittel, bestehend aus den üblichen bekannten Bestandteilen eines Zahnpflegemittels, **dadurch gekennzeichnet,**
   daß es anstelle von bekannten Abrasivkomponenten als Putzkörper Cellulosepulver mit den folgenden Merkmalen:

| Struktur | Pulver | |
|---|---|---|
| Teilchengröße in µm | 40 - 150 | |
| Schüttgewicht bei Absackung g/l | 220 - 230 | |
| pH-Wert | 5,0 - 7,5 | |
| Brechungsindex | 1,55 | |
| Dichte g/cm$^3$ | ca. 1,5 | |
| Trocknungsverlust 2 h/105 °C | < 6 | % |
| Glührückstand 2 h/850 °C | < 0,3 | % |
| wasserlösliche Bestandteile | < 1,0 | % |
| ätherlösliche Bestandteile | < 0,15 | % |
| Calcium-Ionen | < 0,05 | % |
| Chlorid-Ionen | < 0,05 | % |
| Sulfat-Ionen | < 0,05 | % |
| Schwermetall-Ionen als Pb | < 10 ppm | |
| Zn | < 2 ppm | |
| As | < 1 ppm | |
| Cr | < 1 ppm | |
| Fe | < 10 ppm | |
| Cu | < 1 ppm | |

enthält.

2. Zahnpflegemittel nach Anspruch 1,
**dadurch gekennzeichnet,**
daß das Cellulosepulver in einer Menge von 1 bis 25 Gew.-%, vorzugsweise von 10 bis 20 Gew.-%, enthalten ist.

**Claims**

1. Dental care agent, comprising the conventional known constituents of a dental care agent, characterized

11

in that it comprises, instead of known abrasive components, as the cleaning agent cellulose powder having the following features:

| structure | powder |
|---|---|
| particle size in µm | 40 – 150 |
| bulk density when bagged, g/l | 220 – 230 |
| pH value | 5.0 – 7.5 |
| refractive index | 1.55 |
| density, $g/cm^3$ | approx. 1.5 |
| drying loss, 2 h/105°C | < 6 % |
| annealing residue, 2 h/850 °C | < 0.3 % |
| water-soluble constituents | < 1.0 % |
| ether-soluble constituents | < 0.15 % |
| calcium ions | < 0.05 % |
| chloride ions | < 0.05 % |
| sulphate ions | < 0.05 % |
| heavy metal ions, as Pb | < 10 ppm |
| Zn | < 2 ppm |
| As | < 1 ppm |
| Cr | < 1 ppm |
| Fe | < 10 ppm |
| Cu | < 1 ppm. |

2. Dental care agent according to Claim 1, characterized in that the cellulose powder is in a quantity of from 1 to 25 % by weight, preferably from 10 to 20 % by weight.

**Revendications**

1. Composition pour le traitement dentaire constituée des composants habituellement connus d'un dentifrice, caractérisée en ce qu'à la phase des composants abrasifs connus, elle contient comme corps nettoyant de la poudre de cellulose avec les caractéristiques suivantes :

| Structure | poudre |
|---|---|
| Granulométrie en micron env. | 40 - 150 |
| Masse volumique apparente à l'ensachage g/l | 220 - 230 |
| pH | 5,0 - 7,5 |
| Indice de réfraction | 1,55 |
| Densité | env. 1,5 |
| Perte au séchage 2 h/105°C | < 6 % |
| Résidu à la calcination 2 h/850°C | < 0,3 % |
| Composants hydrosolubles | < 1,0 % |
| Composants solubles dans l'éther | < 0,15 % |
| Ions calcium | < 0,05 % |
| Ions chlorure | < 0,05 % |
| Ions sulfate | < 0,05 % |
| Ions métaux lourds sous forme de Pb | < 10 ppm |
| Zn | < 2 ppm |
| As | < 1 ppm |
| Cr | < 1 ppm |
| Fe | < 10 ppm |
| Cu | < 1 ppm |

2. Composition pour le traitement dentaire, caractérisée en ce que la poudre de cellulose est contenue à une concentration de 1 à 25 % en poids, de préférence de 10 à 20 % en poids.